# EUROPÄISCHE PATENTSCHRIFT

(11) **EP 3 706 668 B1**
(45) Veröffentlichungstag und Bekanntmachung des Hinweises auf die Patenterteilung: **05.05.2021**
(21) Anmeldenummer: 18796934.0
(22) Anmeldetag: 05.11.2018
(51) Int. Cl.: A61F 2/78, A61F 2/80, A61F 2/72, A61B 5/0488

(54) **PROTHESENSYSTEM MIT EINEM LINER UND EINEM PROTHESENSCHAFT**
PROSTHESIS SYSTEM WITH A LINER AND A PROSTHESIS SOCKET
SYSTÈME DE PROTHÈSE MUNI D'UNE PELLICULE PROTECTRICE ET D'UNE TIGE DE PROTHÈSE

(30) Priorität: 10.11.2017 DE 102017126462
(43) Veröffentlichungstag der Anmeldung: 16.09.2020
(73) Patentinhaber: Ottobock SE & Co. KGaA, 37115 Duderstadt (DE)
(72) Erfinder: KOPPE, Mario, 99988 Wendehausen (DE)
(74) Vertreter: Gramm, Lins & Partner Patent- und Rechtsanwälte PartGmbB
(86) Internationale Anmeldenummer: PCT/EP2018/080102
(87) Internationale Veröffentlichungsnummer: WO 2019/091891

(56) Entgegenhaltungen:
- US-A- 5 258 037
- US-A1- 2009 132 056
- US-A1- 2013 046 394
- US-A1- 2016 038 314
- US-B1- 8 591 599

## Beschreibung

Die Erfindung betrifft ein Prothesensystem mit einem Liner und einem Prothesenschaft, wie in den Ansprüchen definiert, wobei der Liner ein geschlossenes distales Ende, ein offenes proximales Ende, eine Mantelfläche, die sich von dem distalen Ende zu dem proximalen Ende erstreckt, und eine erste Verbindungseinrichtung aufweist, und wobei der Prothesenschaft eine zweite Verbindungseinrichtung aufweist, die eingerichtet ist mit der ersten Verbindungseinrichtung zusammen zu wirken.

Derartige Prothesensysteme sind in vielen unterschiedlichen Ausgestaltungen aus dem Stand der Technik bekannt. Zum Verwenden des Prothesensystems wird zunächst der Liner über den Amputationsstumpf gezogen, indem er zumeist aufgerollt wird. Anschließend wird der Amputationsstumpf mit dem sich darüber befindende Liner in dem Prothesenschaft eingeführt und an diesem befestigt. Dies kann über Unterdruck geschehen, indem der Zwischenraum zwischen dem Liner und dem Prothesenschaft abgesaugt wird. Für den Fall, dass diese Art der Befestigung nicht ausreicht oder nicht gewünscht ist, sind unterschiedlichste Verbindungseinrichtungen bekannt, die den Liner mit dem Prothesenschaft verbinden können. Aus der US 9,744,056 B2 beispielsweise sind derartige Verbindungseinrichtungen bekannt. Der Liner verfügt an seinem distalen Ende über einen Pin, der in eine dafür im Prothesenschaft vorgesehene Öffnung geführt und arretiert wird. Dabei kann es zu Rastverbindungen oder beispielsweise magnetischen Verbindungen zwischen den Verbindungseinrichtungen kommen. Auch aus der US 9,155,634 B2 ist ein Prothesensystem bekannt, bei dem der Liner in dem Prothesenschaft befestigt wird, indem die erste Verbindungseinrichtung, die am distalen Ende des Liners angeordnet ist, mit der entsprechenden zweiten Verbindungseinrichtung des Prothesenschaftes verbunden wird.

Moderne Liner verfügen heute in vielen Fällen über Elektroden, die beispielsweise in ein elektrisch nicht leitendes Linergrundmaterial eingegossen oder eingeklebt sind. Diese Elektroden können mit der Innenseite des Liners, die dem Amputationsstumpf zugewandt ist, eine bündige Oberfläche bilden oder aus dieser hervorstehen. Sie kommen im angelegten Zustand des Liners mit der Haut des Trägers des Prothesensystems in Kontakt und können so elektrische Signale von der Haut des Trägers abnehmen oder beispielsweise an darunter liegende Muskeln übertragen. Werden elektrische Signale übertragen, ist dies als Muskelstimulation zu Rehabilitationszwecken oder Trainingszwecken von Vorteil. Oftmals werden jedoch myoelektrische Signale, die von den Muskeln ausgesandt werden, über die Elektroden abgenommen und beispielsweise einer Prothesensteuerung zugeführt. Auf diese Weise können Prothesenelemente, beispielsweise eine Prothesenhand, gesteuert werden.

Unabhängig davon, in welche Richtung die elektrischen Signale übertragen werden sollen, muss eine elektrische Verbindung zwischen der Elektrode des Liners und einer elektronischen Datenverarbeitungseinrichtung, die beispielsweise im oder am Prothesenschaft angeordnet sein kann, hergestellt werden. In den genannten Druckschriften US 9,744,056 B2 und US 9,155,634 B2 geschieht diese elektrische Kontaktierung am distalen Ende, da hier ohnehin ein Kontaktbereich zwischen dem Liner und dem Prothesenschaft vorhanden ist. Aus der DE 10 2014 106 070 A1 ist ein Liner bekannt, der eine elektrische Kontaktierung an der Mantelfläche erlaubt. Dazu muss jedoch der Liner in exakt der notwendigen Position am Amputationsstumpf angeordnet werden, um die Kontaktierung in der richtigen Weise sicherzustellen. Ähnliche Ausgestaltungen, bei denen die elektrische Kontaktierung am distalen Ende des Liners erfolgt, sind aus der US 8,591,599 B1, der US 2013/046394 A1 und der US 2016/038314 A1 bekannt.

Aus der WO 2008/040286 A1 ist ein Schaftsystem bekannt, das aus einem Au-ßenschaft und einem Innenschaft besteht. Der Außenschaft verfügt dabei über mehrere Elemente, die relativ zueinander leicht bewegt, beispielsweise aufgebogen werden können. Um eine optimale Passform zu erreichen, ist der Innenschaft an seiner Mantelfläche mit einem mechanischen Verriegelungselement ausgerüstet, das mit einem Gegenelement des Prothesenschaftes zusammenwirken kann und so eine Verbindung der beiden Schaftelemente erlaubt.

Problematisch ist bei der Verwendung von Linern, dass diese oftmals mit einem rotationssymmetrischen Querschnitt ausgerüstet sind und somit in einer Vielzahl unterschiedlicher Positionen am Amputationsstumpf angeordnet werden können. Um myoelektrische Signale von der gewünschten Stelle abnehmen oder auf die gewünschten Muskeln aufbringen zu können, ist es jedoch notwendig, dass der Liner genauso am Amputationsstumpf angeordnet wird, dass die Elektroden in der gewünschten und notwendigen Position am Amputationsstumpf anliegen.

Der Erfindung liegt der daher die Aufgabe zugrunde, ein Prothesensystem so weiterzuentwickeln, dass diese Vorgaben möglichst einfach erreicht werden.

Die Erfindung löst die gestellte Aufgabe durch ein Prothesensystem beinhaltend einen Liner gemäß Anspruch 1 und einen Prothesenschaft gemäß Anspruch 6 das sich dadurch auszeichnet, dass die erste Verbindungseinrichtung an der Mantelfläche nach radial hervorstehend angeordnet ist und die erste Verbindungseinrichtung und die zweite Verbindungseinrichtung eingerichtet sind, eine mechanische Verriegelung und einen elektrischen Kontakt zwischen dem Liner und dem Prothesenschaft herzustellen.

Anders als bei den Vorrichtungen aus dem Stand der Technik ist die erste Verbindungseinrichtung an der Mantelfläche des Liners, also zwischen dem distalen Ende und dem proximalen Ende angeordnet. Dies betrifft insbesondere den Bereich, in dem der Liner einen ringförmigen Querschnitt aufweist. Am distalen Ende befindet sich häufig eine Linerkappe oder Linertasse, die senkrecht zur Längserstreckung des Liners geschnitten, nur einen kreisförmigen, massiven Querschnitt aufweist. Die Längserstreckung oder Längsrichtung eines Liners erstreckt sich vom distalen Ende zum proximalen Ende. Durch die Positionierung der ersten Verbindungseinrichtung an dieser Mantelfläche wird eine Reihe von Vorteilen erreicht. Zunächst ist für den Träger des Prothesensystems bereits beim Anlegen des Liners gut zu erkennen, ob der Liner in der richtigen Orientierung am Amputationsstumpf angeordnet werden kann. Durch die an der Außenseite des Liners angeordnete erste Verbindungseinrichtung, die nach radial hervorsteht, hat der Träger einen guten Anhaltspunkt für diese so wichtige richtige Ausrichtung des Liners. Damit die zweite Verbindungseinrichtung, die am Prothesenschaft angeordnet ist, mit der so angeordneten ersten Verbindungseinrichtung zusammenwirken kann, muss sie auf der gleichen Höhe, also ebenfalls nicht im distalen Bereich, des Prothesenschaftes angeordnet werden. Dies ist insbesondere dann von Vorteil, wenn der Träger des Prothesensystems über einen langen Amputationsstumpf verfügt.

In diesem Fall ist es möglich, dass sich distal des Liners nicht ausreichend Bauraum befindet, um die entsprechenden Verbindungseinrichtungen anzuordnen. Auch dieses Problem wird durch die erfindungsgemäße Ausgestaltung gelöst.

Da durch die erste Verbindungseinrichtung des Liners und die zweite Verbindungseinrichtung des Prothesenschaftes auch der elektrische Kontakt zwischen Liner und Prothesenschaft hergestellt wird, ist es nicht notwendig, die elektrischen Signale in den distalen Bereich des Amputationsstumpfes und des Liners zu leiten und von dort in den Prothesenschaft zu überführen. Anschließend müssten sie zu einer erneut gegebenenfalls nicht im distalen Bereich des Prothesenschaftes angeordneten elektronischen Datenverarbeitungseinrichtung geleitet werden. Der Prothesenliner kann mit Elektroden und/oder Sensoren zum Erfassen von Biosignalen versehen sein. Biosignale sind üblicherweise beispielsweise myoelektrische Signale und/oder Impedanz-Signale, insbesondere Bio-Impedanz-Signale. Zusätzlich oder alternativ dazu kann der Liner wenigstens eine, vorzugsweise mehrere Stimulationselektroden aufweisen, so dass Signale an den Amputationsstumpf und die Haut des Trägers des Liners gesendet werden können.

Vorteilhafterweise weisen die erste Verbindungseinrichtung und die zweite Verbindungseinrichtung zueinander korrespondierende Formschlusselemente auf. Dies können beispielsweise Rastelemente und korrespondierende Hinterschnittelemente sein. Die Rastelemente, die entweder Teil der ersten Verbindungseinrichtung oder Teil der zweiten Verbindungseinrichtung sein können, werden beim Einführen des Liners in den Prothesenschaft so relativ zu den Hinterschnittelementen, die Teil der jeweils anderen Verbindungseinrichtung sind, bewegt, dass sie hinter die Hinterschneidungselemente schnappen und so eine Verriegelung herbeiführen. Selbstverständlich sind auch andere Formschlusselemente möglich.

Vorteilhafterweise werden der Liner und der Prothesenschaft durch die mechanische Verriegelung der beiden Verbindungseinrichtungen sowohl gegen eine Rotationsbewegung als auch gegen eine Translationsbewegung gegeneinander verriegelt. Dadurch wird einerseits verhindert, dass der Prothesenschaft sich relativ zum Liner in einer Rotationsbewegung um den Amputationsstumpf herum bewegt und andererseits ausgeschlossen, dass der Liner beispielsweise wenn der Prothesenschaft unter Zug gerät, aus dem Prothesenschaft herausgezogen werden kann.

Vorzugsweise verfügt der Liner über wenigstens eine Elektrode, die mit der ersten Verbindungseinrichtung durch wenigstens einen elektrischen Leiter verbunden ist. Besonders bevorzugt verfügt der Liner über mehrere Elektroden, beispielsweise zwei, drei oder vier, die jeweils einen elektrischen Leiter aufweisen, der mit der Verbindungseinrichtung verbunden ist. Insbesondere in diesem Fall ist es sinnvoll, mehrere elektrische Kontakte herzustellen, um die Elektroden einzeln anzusteuern und die von den Elektroden abgenommenen Signale einzeln verarbeiten zu können.

In einer bevorzugten Ausgestaltung verfügt der Prothesenschaft über eine elektronische Datenverarbeitungseinrichtung oder ist mit einer solchen verbindbar, die durch den elektrischen Kontakt mit der wenigstens einen Elektrode verbindbar ist. Alternativ oder zusätzlich dazu verfügt der Liner über eine elektronische Datenverarbeitungseinrichtung und über den elektrischen Kontakt wird lediglich eine Stromversorgung hergestellt. In diesem Fall verfügt der Prothesenschaft über eine entsprechende Energiequelle, beispielsweise einen Akku oder eine Batterie, durch die die Stromversorgung der elektronischen Datenverarbeitungseinrichtung, die Teil des Liners oder Teil des Prothesenschaftes sein kann, sichergestellt wird.

Vorteilhafterweise ist ein erster Durchmesser des Liners am distalen Ende in einer ersten Richtung größer als ein zweiter Durchmesser in einer zweiten Richtung, die orthogonal zu dieser ersten Richtung verläuft. Besonders bevorzugt ist der erste Durchmesser der maximale Durchmesser. Es hat sich als besonders vorteilhaft herausgestellt, wenn in diesem Fall der zweite Durchmesser der minimale Durchmesser ist. Derartige Liner sind insbesondere aber nicht ausschließlich für Unterarmamputierte von Vorteil. Während ein Liner für einen Oberschenkelamputierten im Wesentlichen einen kreisförmigen Querschnitt aufweisen kann, da auch der Querschnitt eines Oberschenkels nahezu kreisförmig ausgebildet ist, ist dies bei Unterarmen deutlich anders. Zudem wird durch die Konturierung des Liners in dieser Form bereits eine Verdrehsicherheit und eine Anleghilfe bereitgestellt. Der Querschnitt des Liners kann oval, unregelmäßig oder viereckig ausgebildet sein, wobei bei einer viereckigen Ausgestaltung die Ecken abgerundet sein sollten. Vorteilhafterweise ist die erste Verbindungseinrichtung in Verlängerung des zweiten Durchmessers an der Außenseite angeordnet.

Es hat sich als vorteilhaft herausgestellt, wenn ein Verhältnis des ersten Durchmessers zu dem zweiten Durchmesser zwischen dem distalen Ende und dem proximalen Ende variiert. Dies bedeutet, dass an unterschiedlichen Stellen des Liners entlang seiner Längserstreckung, also vom distalen Ende zum proximalen Ende, unterschiedliche Verhältnisse der entsprechenden Durchmesser vorliegen. Auf diese Weise kann den individuellen Gegebenheiten besonders einfach Rechnung getragen werden.

Die Erfindung löst die gestellte Aufgabe zudem durch einen Liner für ein entsprechendes Prothesensystem und durch einen Prothesenschaft für ein derartiges Prothesensystem wie in den Ansprüchen definiert.

Mit Hilfe der beiliegenden Zeichnungen werden nachfolgend die Ausführungsbeispiele der vorliegenden Erfindung näher erläutert. Es zeigt:
- Fig. 1: - die schematische Darstellung durch einen Teil eines Prothesensystems gemäß einem Ausführungsbeispiel der vorliegenden Erfindung,
- Fig. 2: - die schematische Schnittdarstellung durch einen Teil eines Prothesensystems gemäß einem anderen Ausführungsbeispiel,
- Fig. 3a und 3b: - zwei unterschiedliche Ausführungsbeispiele eines Liners,
- Fig. 4: - die schematische Ansicht und Schnittdarstellung durch einen Liner für ein Prothesensystem gemäß einem Ausführungsbeispiel der vorliegenden Erfindung und
- Fig. 5 und 6: - Schnittdarstellungen durch Prothesensysteme gemäß Ausführungsbeispielen der vorliegenden Erfindung.

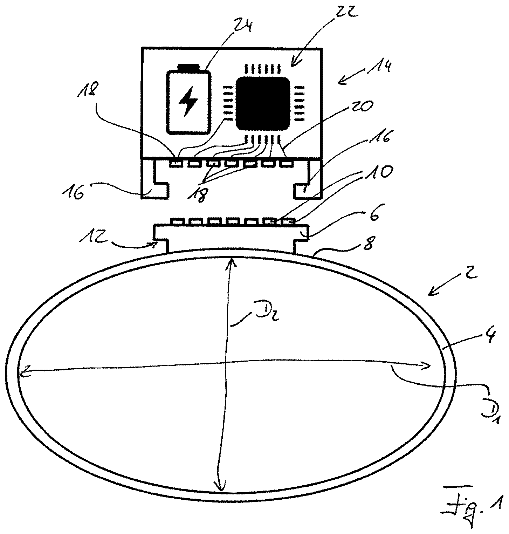

Figur 1 zeigt einen Liner 2 in einer Schnittdarstellung. Gezeigt ist die Mantelfläche 4 in einer Schnittdarstellung. Der Liner verfügt nicht über einen kreisförmigen Querschnitt, sondern über einen ersten Durchmesser D₁ und einen senkrecht dazu stehenden zweiten Durchmesser D₂, der kleiner ist als der erste Durchmesser D₁. In Verlängerung des zweiten Durchmessers D₂ ist eine erste Verbindungseinrichtung 6 an einer Außenseite 8 des Liners 2 angeordnet. Sie verfügt über elektrische Kontakte 10 und über zwei Vorsprünge 12, durch die der elektrische Kontakt und die mechanische Verriegelung erreicht werden können.

Teil eines nicht dargestellten Prothesenschaftes ist eine zweite Verbindungseinrichtung 14, die über zwei Hakenelemente 16 verfügt, die mit den Vorsprüngen 12 der ersten Verbindungseinrichtung 6 zusammenwirken. Sind die Haken 16 mit den Vorsprüngen 12 in Eingriff gebracht, liegen elektrische Kontakte 18 der zweiten Verbindungseinrichtung an den elektrischen Kontakten 10 der ersten Verbindungseinrichtung 8 und die elektrische Verbindung ist hergestellt. Über elektrische Leiter 20 sind die elektrischen Kontakte 18 mit einer elektronischen Datenverarbeitungseinrichtung 22 verbunden, die schematisch dargestellt ist. Sie wird über eine Energiequelle 24 mit elektrischer Energie gespeist.

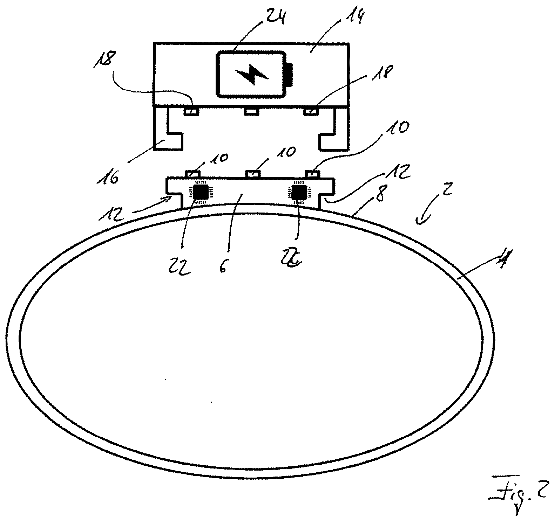

Figur 2 zeigt eine alternative Ausgestaltung. Die Mantelfläche 4 des Liners 2 verfügt wieder nicht über einen kreisförmigen Querschnitt. An der ersten Verbindungseinrichtung 6, die wieder an der Außenseite 8 des Liners 2 angeordnet ist, sind wieder elektrische Kontakte 10 angeordnet, die nun jedoch in deutlich geringer Zahl vorliegen. Sie können mit den elektrischen Kontakten 18 an der zweiten Verbindungseinrichtung 14 in Kontakt gebracht werden, wenn die Hakenelemente 16 mit den Vorsprüngen 12 der ersten Verbindungseinrichtung 6 in Eingriff gebracht werden. Die zweite Verbindungseinrichtung 14 verfügt über die Energiequelle 24, durch die die elektronische Datenverarbeitungseinrichtung 22, die in diesem Ausführungsbeispiel Teil der ersten Verbindungseinrichtung 6 ist, mit Strom versorgt wird.

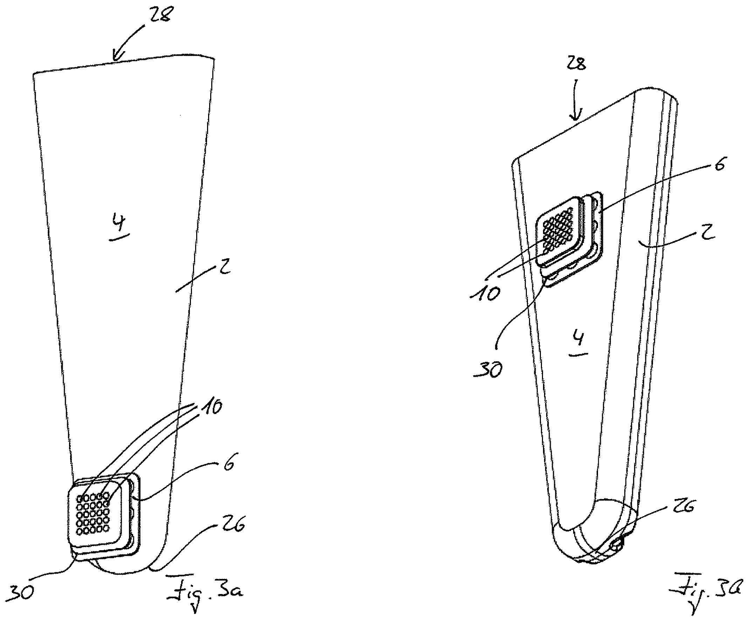

Figuren 3a und 3b zeigen zwei Liner 2 für ein Prothesensystem der hier beschriebenen Art. Sie verfügen über ein geschlossenes distales Ende 26 sowie ein offenes proximales Ende 28. Dazwischen erstreckt sich die Mantelfläche 4. An der Mantelfläche 4 ist die erste Verbindungseinrichtung 6 angeordnet, die mehrere elektrische Kontakte 10 aufweist, die in Form eines Quadratmusters angeordnet sind. Über Hinterschnittelemente 30 kann die Verbindung zu einer nicht dargestellten zweiten Verbindungseinrichtung 14 hergestellt werden.

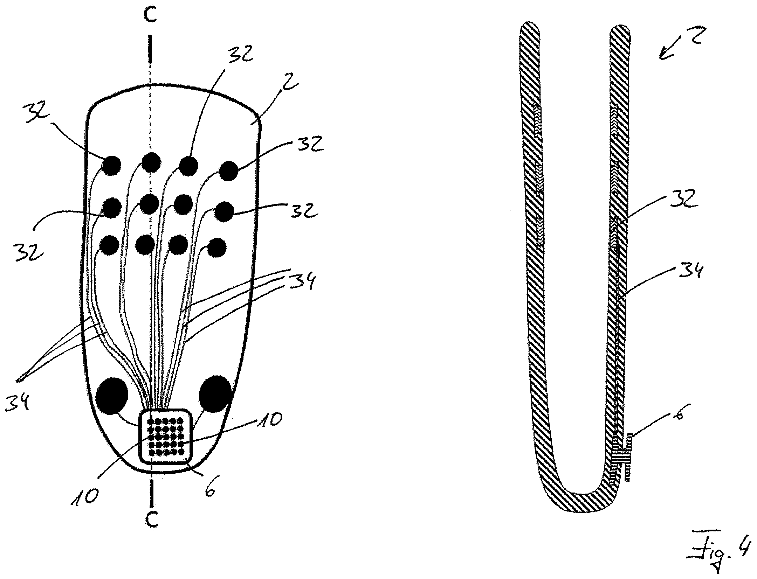

Figur 4 zeigt in der linken Darstellung einen Liner 2 für ein Ausführungsbeispiel der vorliegenden Erfindung. Er verfügt über mehrere Elektroden 32, die jeweils durch einen elektrischen Leiter 34 mit den elektrischen Kontakten 10 der ersten Verbindungseinrichtung 6 verbunden sind. Die elektrischen Leiter 34 verlaufen innerhalb des Materials des Liners 2. In der rechten Darstellung der Figur 4 ist die Schnittdarstellung entlang der Linie C-C dargestellt. Man erkennt den Liner 2 mit den elektrischen Leitern 34 innerhalb des Linermaterials, die zu den Elektroden 32 führen und mit der ersten Verbindungseinrichtung 6 verbunden sind.

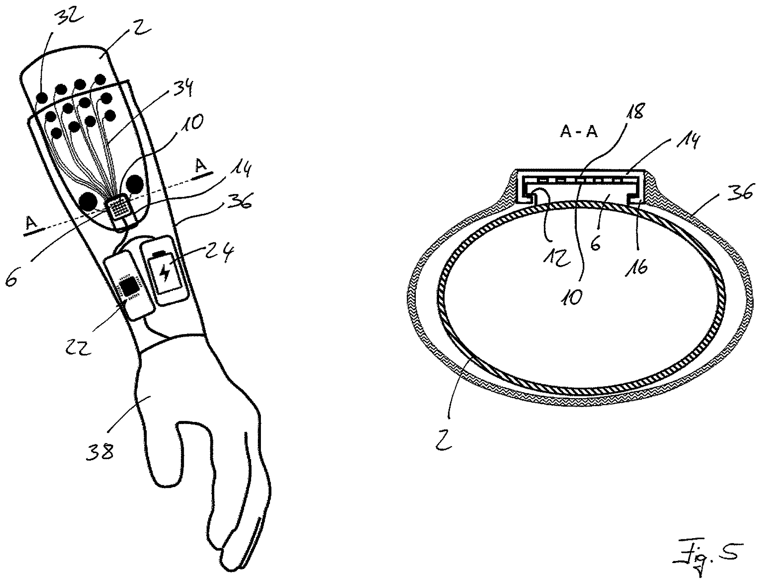

Figur 5 zeigt links eine schematische Ansicht und rechts eine Schnittdarstellung durch ein Prothesensystem gemäß einem Ausführungsbeispiel der vorliegenden Erfindung. Über einen nicht dargestellten Amputationsstumpf befindet sich der Liner 2 ähnlich dem in Figur 4 dargestellten. Der Liner 2 verfügt über Elektroden 32, die über elektrische Leiter 34 mit den elektrischen Kontakten 10 der ersten Verbindungseinrichtung 6 verbunden sind. Darüber befindet sich ein Prothesenschaft 36, an dem sich eine Prothesenhand 38 befindet. Der Prothesenschaft 36 ist transparent dargestellt, um die elektronische Datenverarbeitungseinrichtung 22 sowie die Energiequelle 24, die im Innern des Prothesenschaftes 36 angeordnet und mit der zweiten Verbindungseinrichtung 14 verbunden sind, sichtbar zu machen. Herkömmlicherweise ist der Prothesenschaft 36 nicht transparent ausgebildet.

Figur 5 zeigt in der rechten Darstellung einen Schnitt entlang der Linie A-A. Man erkennt den Liner 2, den Grundkörper des Prothesenschaftes 36 sowie die erste Verbindungseinrichtung 6 und die zweite Verbindungseinrichtung 14. Die Hakenelemente 16 der zweiten Verbindungseinrichtung 14 greifen hinter die Vorsprünge 12 der ersten Verbindungseinrichtung 6 und sorgen so für eine mechanische Verriegelung. Gleichzeitig kommen die elektrischen Kontakte 10 der ersten Verbindungseinrichtung 6 mit den elektrischen Kontakten 18 der zweiten Verbindungseinrichtung 14 in Kontakt.

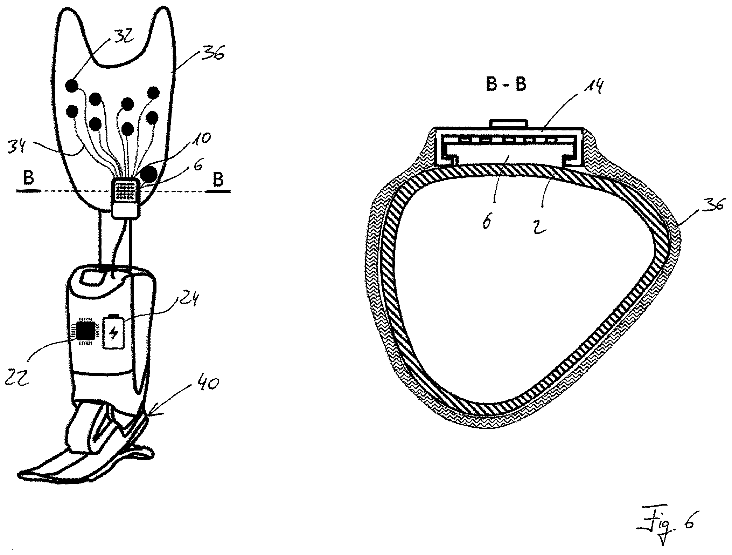

Figur 6 zeigt ein Prothesensystem gemäß einem weiteren Ausführungsbeispiel der vorliegenden Erfindung. Links ist in Figur 6 eine schematische Ansicht dargestellt, in der ein Prothesenschaft 36 dargestellt ist, in dem sich ein nicht dargestellter Liner 2 befindet. Vom Liner sind lediglich die Elektroden 32 sowie die elektrischen Leiter 34 dargestellt, mit denen die Elektroden 32 mit den elektrischen Kontakten 10 der ersten Verbindungseinrichtung 6 verbunden sind. Der Liner selbst ist aus Übersichtlichkeitsgründen nicht dargestellt. Am distalen, also unteren Ende des Prothesenschaftes 36 ist ein Prothesenfuß 40 angeordnet. An einem Unterschenkelelement, das zwischen den Prothesenfuß 40 und dem Prothesenschaft 36 angeordnet ist, befindet sich die Energiequelle 24 sowie die elektronische Datenverarbeitungseinrichtung 22. Figur 6 zeigt in der rechten Darstellung einen Schnitt entlang der Linie B-B mit dem Liner 2, dem Prothesenschaft 36 sowie der ersten Verbindungseinrichtung 6 und der zweiten Verbindungseinrichtung 14. Auch hier kommt es zwischen den beiden Verbindungseinrichtungen 6, 14 sowohl zu einer mechanischen Verriegelung als auch zum Kontakt der jeweiligen elektrischen Kontakte 10, 18.

Die Protheseneinrichtung, insbesondere in den in Figuren 5 und 6 gezeigten Ausführungsformen, kann weitere Sensoren aufweisen, um beispielsweise Einsatzerfordernisse zu ermitteln. Vorzugsweise besitzt die Protheseneinrichtung kinetische Sensoren, beispielsweise Kraft- oder Momentensensoren, und/oder kinematische Sensoren, beispielsweise Beschleunigungs-, Geschwindigkeits- oder Lagesensoren, und/oder Umweltsensoren, beispielsweise optische Sensoren, Ultraschall, Radar oder Empfänger für RFID-Tags auf.

### Bezugszeichenliste

- D₁: erster Durchmesser
- D₂: zweiter Durchmesser
- 2: Liner
- 4: Mantelfläche
- 6: erste Verbindungseinrichtung
- 8: Außenseite
- 10: elektrischer Kontakt
- 12: Vorsprung
- 14: zweite Verbindungseinrichtung
- 16: Hakenelement
- 18: elektrischer Kontakt
- 20: elektrischer Leiter
- 22: elektronische Datenverarbeitungseinrichtung
- 24: Energiequelle
- 26: distales Ende
- 28: proximales Ende
- 30: Hinterschnittelement
- 32: Elektrode
- 34: elektrischer Leiter
- 36: Prothesenschaft
- 38: Prothesenhand
- 40: Prothesenfuß

## Patentansprüche

1. Ein Liner welcher
- ein geschlossenes distales Ende,
- ein offenes proximales Ende,
- eine Mantelfläche, die sich von dem distalen Ende zu dem proximalen Ende erstreckt, und
- eine erste Verbindungseinrichtung aufweist, die eingerichtet ist mit einer zweiten Verbindungseinrichtung an einem Prothesenschaft zusammenzuwirken, und wobei die erste Verbindungseinrichtung eingerichtet ist mit der zweiten Verbindungseinrichtung eine mechanische Verriegelung und einen elektrischen Kontakt zwischen dem Liner und dem Prothesenschaft herzustellen, **dadurch gekennzeichnet, dass** die erste Verbindungseinrichtung an der Mantelfläche nach radial hervorstehend angeordnet ist.

2. Liner nach Anspruch 1, **dadurch gekennzeichnet, dass** der Liner wenigstens eine Elektrode und/oder wenigstens einen Sensor und/ oder wenigstens eine Stimulationseinrichtung aufweist, die mit der ersten Verbindungseinrichtung durch wenigstens einen elektrischen Leiter verbunden ist.

3. Liner nach Anspruch 1 oder 2, **dadurch gekennzeichnet, dass** ein erster Durchmesser des Liners am distalen Ende in einer ersten Richtung größer ist als ein zweiter Durchmesser in einer zweiten Richtung, die orthogonal zu der ersten Richtung verläuft.

4. Liner nach Anspruch 3, **dadurch gekennzeichnet, dass** die erste Verbindungseinrichtung in Verlängerung des zweiten Durchmessers an der Außenseite angeordnet ist.

5. Liner nach Anspruch 3 oder 4, **dadurch gekennzeichnet, dass** ein Verhältnis des ersten Durchmessers zu dem zweiten Durchmesser zwischen dem distalen Ende und dem proximalen Ende variiert.

6. 2. Ein Prothesenschaft welcher
- eine zweite Verbindungseinrichtung aufweist, die eingerichtet ist mit einer ersten Verbindungseinrichtung an einem Liner zusammenzuwirken, wobei
- die zweite Verbindungseinrichtung eingerichtet ist mit der ersten Verbindungseinrichtung eine mechanische Verriegelung und einen elektrischen Kontakt zwischen dem Liner und dem Prothesenschaft herzustellen, **dadurch gekennzeichnet, dass** die zweite Verbindungseinrichtung dergestalt eingerichtet ist, um mit der ersten an der Mantelfläche des Liners nach radial hervorstehend angeordneten Verbindungseinrichtung zusammenzuwirken.

7. Prothesensystem mit einem Liner nach einem der Ansprüche 1-5 und einem Prothesenschaft nach Anspruch 6.

8. Prothesensystem nach Anspruch 7, **dadurch gekennzeichnet, dass** die erste Verbindungseinrichtung und die zweite Verbindungseinrichtung zueinander korrespondierende Formschlusselemente aufweisen.

9. Prothesensystem nach Anspruch 7 oder 8, **dadurch gekennzeichnet, dass** durch die mechanische Verriegelung der Liner und der Prothesenschaft gegen eine Rotationsbewegung und gegen eine Translationsbewegung verriegelbar sind.

10. Prothesensystem nach einem der Ansprüche 7-9, **dadurch gekennzeichnet, dass** der Prothesenschaft eine elektronische Datenverarbeitungseinrichtung aufweist oder mit einer elektronischen Datenverarbeitungseinrichtung verbindbar ist, die durch den elektrischen Kontakt mit der wenigstens einen Elektrode verbindbar ist.

## Claims

1. A liner that features
- a closed distal end,
- an open proximal end,
- a lateral surface that extends from the distal end to the proximal end,
and
- a first connection device that is configured to engage with a second connection device on a prosthesis socket, and wherein the first connection device is configured to establish a mechanical locking and an electrical contact between the liner and the prosthesis socket with the second connection device, **characterised in that** the first connection device is arranged on the lateral surface such that it protrudes radially outwards.

2. The liner according to claim 1, **characterised in that** the liner comprises at least one electrode and/or at least one sensor and/or at least one stimulation device that is connected to the first connection device by at least one electric conductor.

3. The liner according to claim 1 or 2, **characterised in that** a first diameter of the liner at the distal end is greater in a first direction than a second diameter in a second direction that extends orthogonally to the first direction.

4. The liner according to claim 3, **characterised in that** the first connection device is arranged on the outer side as an extension of the second diameter.

5. The liner according to claim 3 or 4, **characterised in that** a ratio of the first diameter to the second diameter varies between the distal end and the proximal end.

6. A prosthesis socket which
- comprises a second connection device that is configured to engage with a first connection device on a liner, wherein
- the second connection device is configured to establish a mechanical locking and an electrical contact between the liner and the prosthesis socket with the first connection device, **characterised in that** the second connection device is configured to engage with the first connection device that is arranged on the lateral surface of the liner such that it protrudes radially outwards.

7. A prosthesis system with a liner according to one of the claims 1 - 5 and a prosthesis socket according to claim 6.

8. The prosthesis system according to claim 7, **characterised in that** the first connection device and the second connection device comprise positive-locking elements that correspond with one another.

9. The prosthesis system according to claim 7 or 8, **characterised in that** the liner and the prosthesis socket can be locked against a rotational movement and a translational movement by the mechanical locking.

10. The prosthesis system according to one of the claims 7 - 9, **characterised in that** the prosthesis socket comprises or can be connected to an electronic data processing device, which can be connected via the electrical contact to the at least one electrode.

## Revendications

1. Doublure comportant
- une extrémité distale fermée,
- une extrémité proximale ouverte,
- une surface enveloppe qui s'étend de l'extrémité distale à l'extrémité proximale, et
- un premier moyen de liaison conçu pour coopérer avec un second moyen de liaison sur une emboîture de prothèse, le premier moyen de liaison étant conçu pour établir avec le second moyen de liaison un verrouillage mécanique et un contact électrique entre la doublure et l'emboîture de prothèse,
**caractérisée en ce que**
le premier moyen de liaison est disposé sur la surface enveloppe en faisant saillie radialement.

2. Doublure selon la revendication 1,
**caractérisée en ce que** la doublure comprend au moins une électrode et/ou au moins un capteur et/ou au moins un moyen de stimulation relié au premier moyen de liaison par au moins un conducteur électrique.

3. Doublure selon la revendication 1 ou 2,
**caractérisée en ce qu'**un premier diamètre de la doublure à l'extrémité distale dans une première direction est supérieur à un second diamètre dans une seconde direction orthogonale à la première direction.

4. Doublure selon la revendication 3,
**caractérisée en ce que** le premier moyen de liaison est disposé sur la face extérieure en prolongement du second diamètre.

5. Doublure selon la revendication 3 ou 4,
**caractérisée en ce qu'**un rapport du premier diamètre sur le second diamètre varie entre l'extrémité distale et l'extrémité proximale.

6. Emboîture de prothèse
- comportant un second moyen de liaison conçu pour coopérer avec un premier moyen de liaison sur une doublure,
- le second moyen de liaison étant conçu pour établir avec le second moyen de liaison un verrouillage mécanique et un contact électrique entre la doublure et l'emboîture de prothèse,
**caractérisée en ce que** le second moyen de liaison est conçu de manière à coopérer avec le premier moyen de liaison disposé sur la surface enveloppe de la doublure en faisant saillie radialement.

7. Système de prothèse comportant une doublure selon l'une des revendications 1 à 5 et une emboîture de prothèse selon la revendication 6.

8. Système de prothèse selon la revendication 7,
**caractérisé en ce que** le premier moyen de liaison et le second moyen de liaison présentent des éléments de coopération de forme qui correspondent l'un à l'autre.

9. Système de prothèse selon la revendication 7 ou 8,
**caractérisé en ce que** la doublure et l'emboîture de prothèse peuvent être verrouillées par le verrouillage mécanique à l'encontre d'un mouvement en rotation et d'un mouvement en translation.

10. Système de prothèse selon l'une des revendications 7 à 9,
**caractérisé en ce que** l'emboîture de prothèse comprend un moyen électronique de traitement de données ou peut être connectée à un moyen électronique de traitement de données qui peut être connecté à ladite au moins une électrode par le contact électrique.
